# EUROPEAN PATENT APPLICATION

(11) **EP 4 786 198 A1**
(43) Date of publication of application: **05.08.2026**
(21) Application number: 24871734.0
(22) Date of filing: 30.08.2024
(51) Int. Cl.: B41M 5/00, B41J 2/01, C09D 11/54, C09D 11/322, D06P 5/00, D06P 5/30

(54) **PRETREATMENT LIQUID FOR TEXTILE PRINTING, INK SET FOR TEXTILE PRINTING, AND TEXTILE PRINTING METHOD**

(30) Priority: 28.09.2023 JP 2023168522
(71) Applicant: FUJIFILM Corporation, Tokyo 106-8620 (JP)
(72) Inventor: IKOSHI, Masao, Ashigarakami-gun Kanagawa 2588577 (JP); SUZUKI, Shota, Ashigarakami-gun Kanagawa 2588577 (JP); MATSUMOTO, Yuka, Ashigarakami-gun Kanagawa 2588577 (JP)
(74) Representative: HGF
(86) International application number: PCT/JP2024/031332
(87) International publication number: WO 2025/069934

(57) **Abstract**

Provided are a pretreatment liquid for textile printing, an ink set for textile printing, and a textile printing method, which is capable of obtaining a printed article having excellent washing fastness, favorable texture, and excellent image quality.

The present invention relates to a pretreatment liquid for textile printing, containing a quaternary ammonium cation represented by Formula (1), having a formula weight of 100 to 300, in which a content of the quaternary ammonium cation represented by Formula (1) is 5% by mass to 30% by mass with respect to the total amount of the pretreatment liquid for textile printing, and the pretreatment liquid for textile printing is used before image recording on a fabric using an ink for textile printing, containing a pigment.

## Description

### BACKGROUND OF THE INVENTION

### 1. Field of the Invention

The present disclosure relates to a pretreatment liquid for textile printing, an ink set for textile printing, and a textile printing method.

### 2. Description of the Related Art

In recent years, various studies have been conducted on a textile printing method onto a fabric.

For example, JP2020-105326A discloses an ink set for ink jet textile printing, which contains an ink composition and a treatment liquid composition, in which the treatment liquid composition contains a cationic compound and a compound having one quaternary ammonium in a molecule, and the ink composition contains a pigment and an organic solvent, and contains 10.0% by mass or more and 55.0% by mass or less of a polyhydric alcohol-based solvent as the organic solvent.

JP2021-70880A discloses an ink jet recording method of jetting a white ink composition for ink jet textile printing, containing a white pigment and water, using an ink jet recording device and recording the ink composition on a fabric, in which the white ink composition is jetted as a plurality of liquid droplets of 9 ng or less from the same nozzle, and the plurality of liquid droplets are jetted and recorded such that the plurality of liquid droplets coalesce before landing on the cloth by making a flight speed of a liquid droplet jetted later faster than a flight speed of a liquid droplet jetted earlier among the plurality of liquid droplets.

### SUMMARY OF THE INVENTION

However, in some cases, there is a demand for a printed article obtained by textile printing on a fabric, which has excellent washing fastness, favorable texture, and further improved image quality.

The present disclosure has been made in view of such circumstances, and an object to be achieved by one aspect of the present disclosure is to provide a pretreatment liquid for textile printing, an ink set for textile printing, and a textile printing method, which is capable of obtaining a printed article having excellent washing fastness, favorable texture, and excellent image quality.

The present disclosure includes the following aspects.
<1> A pretreatment liquid for textile printing, comprising:
   a quaternary ammonium cation represented by Formula (1), having a formula weight of 100 to 300;
   in which a content of the quaternary ammonium cation represented by Formula (1) is 5% by mass to 30% by mass with respect to a total amount of the pretreatment liquid for textile printing, and
   the pretreatment liquid for textile printing is used before image recording on a fabric using an ink for textile printing, containing a pigment, 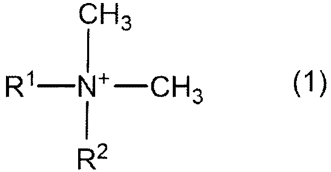
   in Formula (1), both R¹ and R² are a group having 10 or less carbon atoms.
<2> The pretreatment liquid for textile printing according to <1>,
   in which R¹ and R² are each independently an unsubstituted alkyl group, a substituted alkyl group, a vinyl group, an aryl group, an allyl group, an alkyl ester group, a (meth)acryloyl group, or an amino group, each having 10 or less carbon atoms.
<3> The pretreatment liquid for textile printing according to <1> or <2>, further comprising:
   an alkanediol,
   in which a content of the alkanediol is 3% by mass to 30% by mass with respect to the total amount of the pretreatment liquid for textile printing.
<4> The pretreatment liquid for textile printing according to any one of <1> to <3>,
   in which the quaternary ammonium cation represented by Formula (1) is at least one selected from the group consisting of a diallyldimethylammonium cation, a (2-hydroxyethyl)trimethylammonium cation, and an N-(2-acryloyloxyethyl)-N-benzyl-N,N-dimethylammonium cation.
<5> The pretreatment liquid for textile printing according to any one of <1> to <4>,
   in which the formula weight of the quaternary ammonium cation represented by Formula (1) is 100 to 200.
<6> An ink set for textile printing, comprising:
   the pretreatment liquid for textile printing according to any one of <1> to <5>; and
   an ink for textile printing, containing a pigment.
<7> The ink set for textile printing according to <6>,
   in which the ink for textile printing contains an alkanediol, and
   a content of the alkanediol is 3% by mass to 30% by mass with respect to a total amount of the ink for textile printing.
<8> The ink set for textile printing according to <6> or <7>,
   in which at least one of the pretreatment liquid for textile printing or the ink for textile printing contains an alkanediol, and
   in a case where the pretreatment liquid for textile printing and the ink for textile printing have the same mass,
      an effective cation amount in the pretreatment liquid for textile printing is defined as A mmol/L, and
      a total content of the alkanediol contained in the pretreatment liquid for textile printing and the ink for textile printing is defined as B% by mass,
   a ratio of A to B is 15 or more.
<9> The ink set for textile printing according to any one of <6> to <8>,
   in which the ink for textile printing further contains an alkylene glycol alkyl ether, and
   a content of the alkylene glycol alkyl ether is 2% by mass to 20% by mass with respect to a total amount of the ink for textile printing.
<10> The ink set for textile printing according to any one of <6> to <9>,
   in which the ink for textile printing further contains urethane resin particles.
<11> The ink set for textile printing according to any one of <6> to <10>,
   in which the pigment is dispersed by a polymer having a crosslinking structure.
<12> A textile printing method comprising:
   a pretreatment liquid applying step of applying the pretreatment liquid for textile printing, which is contained in the ink set for textile printing according to any one of <6> to <11>, onto a fabric; and
   an ink applying step of applying the ink for textile printing, which is contained in the ink set for textile printing according to any one of <6> to <11>, onto the fabric to which the pretreatment liquid for textile printing is applied.
<13> The textile printing method according to <12>,
   in which, in the pretreatment liquid applying step, the pretreatment liquid for textile printing is applied by an ink jet recording method.
<14> The textile printing method according to <13>,
   in which the ink jet recording method is performed in a single pass.
<15> The textile printing method according to <12>, further comprising:
   a step of heating the fabric to which the ink for textile printing is applied at a temperature of 100°C or higher, after the ink applying step.
<16> The textile printing method according to any one of <12> to <15>,
   in which the textile printing method does not comprise a step of washing the fabric to which the ink for textile printing is applied, after the ink applying step.
<17> The textile printing method according to any one of <12> to <16>,
   in which the fabric is in roll form and is made of cotton or a cotton-polyester blend.

According to the aspect of the present disclosure, it is possible to provide a pretreatment liquid for textile printing, an ink set for textile printing, and a textile printing method, which is capable of obtaining a printed article having excellent washing fastness, favorable texture, and excellent image quality.

### BRIEF DESCRIPTION OF THE DRAWINGS

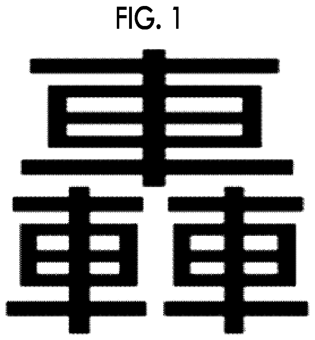

FIG. 1 is a diagram for describing details of an evaluation standard of image quality in Examples.

### DESCRIPTION OF THE PREFERRED EMBODIMENTS

Hereinafter, the pretreatment liquid for textile printing, the ink set for textile printing, and the textile printing method according to the present disclosure will be described in detail.

In the present specification, the numerical ranges shown using "to" means ranges including the numerical values described before and after "to" as the minimum value and the maximum value.

In a numerical range described in a stepwise manner in the present specification, an upper limit value or a lower limit value described in a certain numerical range may be replaced with an upper limit value or a lower limit value in another numerical range described in a stepwise manner. In addition, in the numerical range described in the present specification, an upper limit value and a lower limit value described in a certain numerical range may be replaced with values shown in Examples.

In the present specification, in a case where a plurality of substances corresponding to each component in a composition is present, the amount of each component in the composition means the total amount of the plurality of substances present in the composition, unless otherwise specified.

In the present specification, a combination of two or more preferred aspects is a more preferred aspect.

In the present specification, the meaning of the term "step" includes not only an independent step but also a step whose intended purpose is achieved even in a case where the step is not clearly distinguished from other steps.

### [Pretreatment liquid for textile printing]

The pretreatment liquid for textile printing (hereinafter, also referred to as "pretreatment liquid") according to the present disclosure contains a quaternary ammonium cation represented by Formula (1), having a formula weight of 100 to 300.

Hereinafter, the quaternary ammonium cation represented by Formula (1) is also referred to as "quaternary ammonium cation (1)".

A content of the quaternary ammonium cation (1) is 5% by mass to 30% by mass with respect to the total amount of the pretreatment liquid.

The pretreatment liquid according to the present disclosure is for textile printing. Specifically, the pretreatment liquid according to the present disclosure is a pretreatment liquid used before image recording on a fabric using an ink for textile printing, containing a pigment. That is, the pretreatment liquid and the ink for textile printing are applied onto the fabric in this order.

In a case where the pretreatment liquid according to the present disclosure is applied onto a fabric and then the ink is applied onto the fabric to which the pretreatment liquid is applied, a printed article having excellent washing fastness, favorable texture, and excellent image quality can be obtained.

In the related art, a method of applying a pretreatment liquid containing a large amount of a cationic polymer onto a fabric in advance has been known. However, in a case where the pretreatment liquid containing a large amount of the cationic polymer is applied onto the fabric, a phenomenon may occur in which texture of a printed article to be obtained is deteriorated.

It is considered that, in a case where the cationic polymer is contained in the pretreatment liquid, the cationic polymer and a dispersant contained in an ink form a crosslinking structure on the fabric, which deteriorates the texture. On the other hand, it is considered that the pretreatment liquid according to the present disclosure contains the quaternary ammonium cation (1) having a formula weight of 100 to 300, and thus the texture is excellent.

In addition, the ink is aggregated by the quaternary ammonium cation (1), and thus washing fastness is excellent. Furthermore, since both R¹ and R² of the quaternary ammonium cation (1) are a group having 10 or less carbon atoms, R¹ and R² are not excessively hydrophobic. Therefore, it is considered that, in a case where the ink for textile printing is applied onto a pretreatment layer formed of the pretreatment liquid, the ink for textile printing is easily spread and thus image quality is excellent.

On the other hand, the pretreatment liquid disclosed in JP2020-105326A contains a betaine compound, and there is no description focusing on the quaternary ammonium cation (1). As the pretreatment liquid disclosed in JP2021-70880A, it is disclosed that a quaternary ammonium cation in which R¹ and R² are more than 10 is used, and the image quality is insufficient.

Hereinafter, each component contained in the pretreatment liquid according to the present disclosure will be described in detail.

### <Quaternary ammonium cation (1)>

The pretreatment liquid contains the quaternary ammonium cation (1) having a formula weight of 100 to 300.

The formula weight of the quaternary ammonium cation (1) is 100 to 300, preferably 100 to 200. In a case where the formula weight is 300 or less, a printed article having excellent texture can be obtained. In addition, in a case where the formula weight is 100 or more, a printed article having excellent washing fastness can be obtained.

The formula weight can be calculated from atomic weights of atoms constituting the quaternary ammonium cation (1) by analyzing a structure of the quaternary ammonium cation contained in the pretreatment liquid using a nuclear magnetic resonance (NMR) method.

The quaternary ammonium cation (1) is represented by Formula (1).

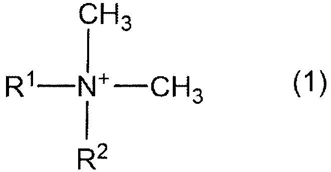

In Formula (1), both R¹ and R² are a group having 10 or less carbon atoms.

In a case where the number of carbon atoms in both R¹ and R² in the quaternary ammonium cation (1) is 10 or less, R¹ and R² are not excessively hydrophobic. Therefore, in a case where the ink for textile printing is applied onto a pretreatment layer formed of the pretreatment liquid, the ink for textile printing is easily spread and thus the image quality is excellent.

From the above-described viewpoint, the number of carbon atoms in R¹ and R² is preferably 10 or less, more preferably 7 or less, and still more preferably 1 to 7.

Among these, R¹ and R² are each independently preferably an unsubstituted alkyl group, a substituted alkyl group, a vinyl group, an aryl group, an allyl group, an alkyl ester group, a (meth)acryloyloxyalkyl group, or an amino group, each having 10 or less carbon atoms; more preferably an unsubstituted alkyl group, a substituted alkyl group, an allyl group, or a (meth)acryloyloxyalkyl group; and still more preferably an unsubstituted alkyl group, a substituted alkyl group, or an allyl group.

The unsubstituted alkyl group having 10 or less carbon atoms may be linear, branched, or cyclic. Among these, the unsubstituted alkyl group having 10 or less carbon atoms is preferably linear. The number of carbon atoms in the unsubstituted alkyl group is preferably 6 or less, more preferably 3 or less, and still more preferably 1 or 2.

The substituted alkyl group having 10 or less carbon atoms refers to an alkyl group having a substituent, in which the number of carbon atoms including carbon atoms of the substituent is 10 or less.

In the substituted alkyl group having 10 or less carbon atoms, the alkyl group may be linear, branched, or cyclic. Among these, in the substituted alkyl group having 10 or less carbon atoms, the alkyl group is preferably linear. The number of carbon atoms in the substituted alkyl group is preferably 6 or less, more preferably 3 or less, and still more preferably 1 or 2.

Examples of the substituent in the substituted alkyl group having 10 or less carbon atoms include a hydroxy group, a carboxy group, an alkyl group, an aryl group, a heteroaryl group, an alkoxy group, an aryloxy group, a heteroaryloxy group, an alkylthioether group, an arylthioether group, a heteroarylthioether group, and a (meth)acryloyl group.

Among these, from the viewpoint of storage stability of the pretreatment liquid, the substituent is preferably a hydroxy group, an aryl group, or a (meth)acryloyl group, and more preferably a hydroxy group.

The quaternary ammonium cation (1) is preferably contained in the pretreatment liquid as a quaternary ammonium salt. Examples of a counteranion in the quaternary ammonium salt include a halide ion such as Cl⁻, Br⁻, and I⁻; an organic sulfonate anion having a substituent selected from an alkyl group, an alkenyl group, an alkynyl group, a cycloalkyl group, an alkoxy group, an aryl group, an aralkyl group, or a heterocyclic group; and PF₆⁻ and BF₄⁻. From the viewpoint of solubility in the pretreatment liquid, the counteranion is preferably Cl⁻, Br⁻, or a sulfonate anion having an alkyl group, more preferably Cl⁻ or Br⁻, and still more preferably Cl⁻.

Among these, from the viewpoint of washing fastness, texture, and image quality, the quaternary ammonium cation (1) is preferably at least one selected from the group consisting of a diallyldimethylammonium cation, a (2-hydroxyethyl)trimethylammonium cation, and an N-(2-acryloyloxyethyl)-N-benzyl-N,N-dimethylammonium cation; and more preferably at least one selected from the group consisting of a diallyldimethylammonium cation and a (2-hydroxyethyl)trimethylammonium cation.

A content of the quaternary ammonium cation (1) is 5% by mass to 30% by mass, more preferably 8% by mass to 25% by mass, and still more preferably 10% by mass to 20% by mass with respect to the total amount of the pretreatment liquid. In a case where the content of the quaternary ammonium cation (1) is 5% by mass or more, a printed article having excellent image quality is obtained. On the other hand, in a case where the content of the quaternary ammonium cation is 30% by mass or less, a printed article having excellent texture is obtained.

The content of the quaternary ammonium cation in the pretreatment liquid can be calculated by, for example, the following method.

First, in a case where water and an aqueous organic solvent, which will be described later, are contained in the pretreatment liquid, the water and the water-based organic solvent are removed by a heat treatment or the like. Next, the quaternary ammonium salt is extracted from the solid content contained in the pretreatment using GPC. A structure of the quaternary ammonium salt is analyzed using the NMR method, and the quaternary ammonium cation constituting the quaternary ammonium salt is specified. An amount of the quaternary ammonium cation is calculated based on the amount of the extracted quaternary ammonium salt. The content of the quaternary ammonium cation in the pretreatment is calculated based on the amount of the pretreatment liquid and the amount of the quaternary ammonium salt.

### (Water)

It is preferable that the pretreatment liquid contains water. A content of water in the pretreatment liquid is preferably 40% by mass or more, more preferably 50% by mass or more, still more preferably 60% by mass or more, and particularly preferably 80% by mass or more with respect to the total amount of the pretreatment liquid. In addition, the content of water is preferably 98% by mass or less and more preferably 95% by mass or less with respect to the total amount of the pretreatment liquid.

### (Organic solvent)

The pretreatment liquid preferably contains at least one organic solvent.

Examples of the organic solvent include an alcohol-based solvent, an amide-based solvent, a nitrile-based solvent, a polyalkylene glycol-based solvent, and a polyalkylene glycol alkyl ether-based solvent.

From the viewpoint of improving jettability, the pretreatment liquid preferably further contains an alkanediol.

Examples of the alkanediol include 2-methyl-1,3-propanediol, 1,3-propanediol, 2,2-dimethyl-1,3-propanediol, 1,4-butanediol, 1,3-butanediol, 1,2-butanediol, 2,3-butanediol, pinacol, ethylene glycol, diethylene glycol, triethylene glycol, tetraethylene glycol, and propylene glycol.

Among these, from the viewpoint of further improving the jettability, the alkanediol is preferably an alkanediol having 1 to 4 carbon atoms.

From the viewpoint of improving the jettability, a proportion of the alkanediol in the organic solvent is preferably 90% by mass or more, and more preferably 90% by mass or more. The upper limit value of the above-described proportion may be 100% by mass.

From the viewpoint of further improving the jettability, the organic solvent preferably consists of only the alkanediol.

A content of the alkanediol is preferably 3% by mass to 30% by mass and more preferably 5% by mass to 20% by mass with respect to the total amount of the pretreatment liquid. In a case where the content of the alkanediol is 3% by mass or more, the effect of improving the jettability is obtained. On the other hand, in a case where the content of the alkanediol is 30% by mass or less, the ink is less likely to bleed, and thus the image quality is improved.

A content of the organic solvent is preferably 3% by mass to 30% by mass and more preferably 5% by mass to 20% by mass with respect to the total amount of the pretreatment liquid.

### (Surfactant)

The pretreatment liquid may contain at least one surfactant.

The surfactant is not particularly limited, and a known surfactant such as a silicone-based surfactant, a fluorine-based surfactant, and an acetylene glycol-based surfactant can be used.

In a case where the pretreatment liquid according to the present disclosure contains a surfactant, a content of the surfactant is preferably 0.05% by mass to 2.0% by mass and more preferably 0.1% by mass to 2.0% by mass with respect to the total amount of the pretreatment liquid.

### (Other aggregating agents)

The quaternary ammonium cation functions as an aggregating agent for aggregating a pigment in the ink. The pretreatment liquid may contain at least one of other aggregating agents, in addition to the quaternary ammonium cation. Examples of the other aggregating agents include a polyvalent metal salt and a cationic compound.

### -Polyvalent metal salt-

The polyvalent metal salt is a compound composed of a metal ion having a valence of 2 or more, and an anion. Specific examples of the polyvalent metal salt include calcium chloride, calcium nitrate, calcium sulfate, calcium acetate, calcium hydroxide, calcium carbonate, magnesium chloride, magnesium acetate, magnesium sulfate, magnesium carbonate, barium sulfate, barium chloride, zinc sulfide, zinc carbonate, and copper nitrate.

### -Cationic compound-

The cationic compound is not particularly limited, and may be a low-molecular-weight compound or a high-molecular-weight compound. The cationic compound does not include a quaternary ammonium salt consisting of the above-described quaternary ammonium cation and a counteranion.

### (Other components)

The pretreatment liquid may contain a component other than the above-described components. Examples of other components include a pH adjusting agent, a fluorescent brightener, a surface tension adjuster, a defoamer, a drying inhibitor, a lubricant, a thickener, an ultraviolet absorber, a fading inhibitor, an antistatic agent, a matting agent, an antioxidant, a resistivity adjuster, a rust inhibitor, a reducing inhibitor, a preservative, a fungicide, and a chelating agent.

### [Ink set for textile printing]

The ink set for textile printing (hereinafter, also referred to as "ink set") according to the present disclosure contains the above-described pretreatment liquid, and an ink for textile printing, containing a pigment.

In a case where the pretreatment liquid in the ink set according to the present disclosure is applied onto a fabric and then the ink composition in the ink set according to the present disclosure is applied onto the fabric to which the pretreatment liquid is applied, a printed article having excellent washing fastness, favorable texture, and excellent image quality can be obtained.

### <Ink>

The ink contains at least one pigment.

A commercially available organic pigment or inorganic pigment may be used as the pigment. Examples of the pigment include pigments described in "Encyclopedia of Pigments" edited by Seishiro Ito (2000), "Industrial Organic Pigments", W. Herbst, K. Hunger, JP2002-12607A, JP2002-188025A, JP2003-26978A, and JP2003-342503A.

In addition, the pigment may be a water-insoluble pigment which can be dispersed in water by a dispersant, or may be a self-dispersing pigment. The self-dispersing pigment is a pigment which can be dispersed in water without using a dispersant. The self-dispersing pigment is, for example, a compound in which at least one selected from the group consisting of a hydrophilic group such as a carbonyl group, a hydroxyl group, a carboxyl group, a sulfo group, and a phosphoric acid group and salts thereof is chemically bonded to a surface of the pigment directly or through another group.

Examples of the organic pigment include an azo pigment, a polycyclic pigment, a chelate dye, a nitro pigment, a nitroso pigment, and aniline black. Examples of the azo pigment include an azo lake, an insoluble azo pigment, a condensed azo pigment, and a chelated azo pigment. Examples of the polycyclic pigment include a phthalocyanine pigment, a perylene pigment, a perinone pigment, an anthraquinone pigment, a quinacridone pigment, a dioxazine pigment, an indigo pigment, a thioindigo pigment, an isoindolinone pigment, and a quinophthalone pigment. Examples of the chelate dye include a basic dye-type chelate, and an acid dye-type chelate.

Examples of the inorganic pigment include titanium oxide, iron oxide, calcium carbonate, barium sulfate, aluminum hydroxide, Barium Yellow, Cadmium Red, Chrome Yellow, and carbon black.

From the viewpoint of image density and jettability of the ink, a content of the pigment in the ink is preferably 1% by mass to 20% by mass, more preferably 1% by mass to 15% by mass, and still more preferably 1% by mass to 10% by mass with respect to the total amount of the ink.

### (Water)

It is preferable that the ink contains water. A content of water is not particularly limited, and is, for example, 40% by mass to 70% by mass.

### (Dispersant)

The ink preferably contains a dispersant in order to disperse the pigment. The dispersant is not particularly limited as long as it is a polymer capable of dispersing the pigment, but from the viewpoint of dispersion stability, it preferably includes at least one hydrophilic group.

The dispersant may be a random copolymer or a block copolymer.

The dispersant may have a crosslinking structure.

Among these, from the viewpoint of improving the jettability, the pigment is preferably dispersed by a polymer having a crosslinking structure.

In a case of preparing the ink, a pigment dispersion liquid in which the pigment is dispersed in water using the dispersant can also be used. As the pigment aqueous dispersion liquid, for example, a pigment dispersion liquid described in JP2012-7148A can be used. In addition, as the pigment dispersion liquid in which the pigment is dispersed in water by the polymer having a crosslinking structure, a commercially available product such as Pro-jet Black APD1000 (manufactured by Fujifilm Imaging Colorants) can also be used.

### (Organic solvent)

The ink preferably contains at least one organic solvent.

Examples of the organic solvent include an alcohol-based solvent, an amide-based solvent, a nitrile-based solvent, a polyalkylene glycol-based solvent, and a polyalkylene glycol alkyl ether-based solvent.

From the viewpoint of improving jettability, the pretreatment liquid preferably further contains an alkanediol.

Among these, from the viewpoint of further improving the jettability, the alkanediol is preferably an alkanediol having 1 to 4 carbon atoms.

A content of the alkanediol is preferably 3% by mass to 30% by mass and more preferably 5% by mass to 20% by mass with respect to the total amount of the pretreatment liquid. In a case where the content of the alkanediol is 3% by mass or more, the effect of improving the jettability is obtained. On the other hand, in a case where the content of the alkanediol is 30% by mass or less, the ink is less likely to bleed, and thus the image quality is improved.

The pretreatment liquid preferably further contains an alkylene glycol alkyl ether.

Examples of the alkylene glycol alkyl ether include ethylene glycol monomethyl ether, ethylene glycol monobutyl ether, diethylene glycol, dipropylene glycol, propylene glycol monomethyl ether, diethylene glycol monomethyl ether, triethylene glycol, polyethylene glycol, propylene glycol monomethyl ether, dipropylene glycol monomethyl ether, tripropylene glycol monomethyl ether, diethylene glycol monobutyl ether, and diethylene glycol monobutyl ether.

From the viewpoint of improving rub resistance, a content of the alkylene glycol alkyl ether is preferably 2% by mass to 20% by mass with respect to the total amount of the ink.

In a case where the content of the alkylene glycol alkyl ether is 2% by mass or more, an effect of improving the rub resistance is obtained. On the other hand, in a case where the content of the alkylene glycol alkyl ether is 20% by mass or less, it is possible to achieve both the rub resistance and drying properties of the image.

A content of the organic solvent is preferably 3% by mass to 40% by mass and more preferably 5% by mass to 30% by mass with respect to the total amount of the pretreatment liquid.

### (Resin particles)

The ink preferably contains at least one type of resin particles.

A resin constituting the resin particles is preferably a water-insoluble resin. The "water-insoluble" in the water-insoluble resin means a property that an amount dissolved in 100 g of distilled water at 25°C is less than 2 g.

A volume average particle diameter of the resin particles is preferably 1 nm to 300 nm, more preferably 3 nm to 200 nm, and still more preferably 5 nm to 150 nm.

In the present disclosure, the volume average particle diameter means a value measured using a laser diffraction/scattering type particle size distribution analyzer.

As the measuring device, a particle size distribution measuring device "MICROTRAC MT-3300II" (manufactured by Nikkiso Co., Ltd.) is exemplified.

Examples of the resin particles include urethane resin particles, acrylic resin particles, and ester resin particles.

For the resin particles, for example, descriptions described in paragraphs 0038 to 0114 of WO2021/192720A and paragraphs 0109 to 0120 of JP2015-25076A may be referred to.

Among these, from the viewpoint of improving the texture of the printed article, the ink preferably contains urethane resin particles.

In a case where the ink contains resin particles, a content of the resin particles is preferably 1% by mass to 20% by mass and more preferably 2% by mass to 15% by mass with respect to the total amount of the ink.

### (Additive)

The ink may contain an additive such as a surfactant, colloidal silica, a co-sensitizer, an ultraviolet absorber, an antioxidant, an antifading agent, a conductive salt, and a basic compound, as necessary.

### (Relationship between effective cation and alkanediol)

It is preferable that at least one of the pretreatment liquid or the ink contains the alkanediol, and from the viewpoint of jettability, it is more preferable that both the pretreatment liquid and the ink contain the alkanediol.

However, in a case where the content of the alkanediol is increased, bleeding is likely to occur, so that it is preferable to maintain a balance with an effective cation in the pretreatment liquid.

Specifically, in a case where the pretreatment liquid and the ink have the same mass, an effective cation amount in the pretreatment liquid is defined as A mmol/L, and a total content of the alkanediol contained in the pretreatment liquid and the ink is defined as B% by mass, a ratio of A to B (that is, A/B) is preferably 15 or more.

The effective cation amount is a value obtained by dividing the content of the quaternary ammonium cation (1) contained in 1 liter of the pretreatment liquid by the formula weight of the quaternary ammonium cation (1) per unit cationic charge.

From the viewpoint of further suppressing the bleeding of the image, A/B is more preferably 10 or more. From the viewpoint of maintaining the texture, the upper limit value of A/B is preferably 100.

### [Textile printing method]

It is preferable that the textile printing method according to the present disclosure includes a pretreatment liquid applying step of applying the pretreatment liquid contained in the above-described ink set onto a fabric, and an ink applying step of applying the ink composition contained in the above-described ink set onto the fabric to which the pretreatment liquid is applied.

In the textile printing method according to the present disclosure, since the ink set according to the present disclosure is used, a printed article having excellent washing fastness, favorable texture, and excellent image quality can be obtained.

### (Pretreatment liquid applying step)

In the textile printing method according to the present disclosure, first, the pretreatment liquid is applied onto a fabric.

### -Fabric-

Examples of fiber types in the fabric include synthetic fibers such as nylon, polyester, and acrylonitrile fibers; semi-synthetic fibers such as acetate and rayon; natural fibers such as cotton, silk, and wool; and mixed fibers consisting of two or more selected from the group consisting of the synthetic fibers, the semi-synthetic fibers, and the natural fibers.

The fiber in the fabric is preferably at least one selected from the group consisting of cotton and polyester. Examples of an aspect of the fabric include woven fabric, knitted fabric, and nonwoven fabric. The fabric may be fabric for a textile product.

In particular, the fabric is preferably in roll form and is made of cotton or a cotton-polyester blend.

Examples of the textile product include clothing items (for example, T-shirts, sweatshirts, jerseys, pants, sweatsuits, dresses, blouses, and the like), bedding, and handkerchiefs.

### -Application of pretreatment liquid-

A method of applying the pretreatment liquid onto the fabric is not particularly limited, and examples thereof include a coating method, a padding method, an ink jet method, a spray method, and a screen printing method. Among these, from the viewpoint of improving the texture of the obtained printed article, the method of applying the pretreatment liquid onto the fabric is preferably an ink jet method. That is, it is preferable to apply the pretreatment liquid by an ink jet recording method.

As the ink jet recording method, a generally known method can be used, and examples thereof include an electric charge control method of jetting the pretreatment liquid by using an electrostatic attraction force; a drop-on-demand method (pressure pulse method) of using a vibration pressure of a piezoelectric element; an acoustic ink jet method of converting an electric signal into an acoustic beam, irradiating the pretreatment liquid, and jetting the pretreatment liquid by using a radiation pressure; and a thermal ink jet method of heating the pretreatment liquid to form air bubbles and utilizing the generated pressure.

In general, an image recording method by an ink jet recording device includes a shuttle scan method (also referred to as a "serial head method") of recording images using a short serial head, and a single-pass method (also referred to as a "line head method") that image recording is performed using a line head in which recording elements are arranged corresponding to the entire width direction of the recording medium. In the shuttle scan method, images are recorded while scanning the serial head in the width direction of the recording medium. On the other hand, in the single-pass method, images can be recorded on the entire surface of the recording medium by scanning the recording medium in the direction orthogonal to the arrangement direction of the recording elements. Therefore, different from the shuttle scan method, the single-pass method does not require a transport system of scanning the serial head, such as a carriage. In addition, in the single-pass method, movement of the carriage and complicated scanning control with the recording medium are not required, and only the recording medium moves, so that the recording speed can be increased as compared with the shuttle scan method.

The ink jet recording method is preferably performed in a single pass. With the textile printing method according to the present disclosure, even in a case of being performed in a single pass, a printed article having excellent washing fastness, favorable texture, and excellent image quality can be obtained.

An amount of droplets of the pretreatment liquid jetted from an ink jet head is preferably 1 picoliter (pL) to 150 pL, more preferably 2 pL to 120 pL, and particularly preferably 20 pL to 60 pL. The amount of droplets jetted means a volume of ink jetted from one nozzle at one time by the ink jet recording method.

From the viewpoint of texture, an application amount of the pretreatment liquid is preferably 10 g/m² to 30 g/m², and more preferably 15 g/m² to 25 g/m².

A resolution of the pretreatment liquid in jetting is preferably 200 dot per inch (dpi) or more × 200 dpi or more, and more preferably 400 dpi or more × 400 dpi or more and 1200 dpi or less × 1200 dpi or less. The "dpi" means the number of dots per 25.4 mm.

### (Ink applying step)

In the textile printing method according to the present disclosure, the ink contained in the ink set is applied onto the fabric to which the pretreatment liquid is applied, after the pretreatment liquid applying step.

### -Application of ink-

A method of applying the ink onto the fabric to which the pretreatment liquid is applied is not particularly limited, and examples thereof include a coating method, a padding method, an ink jet method, a spray method, and a screen printing method. Among these, from the viewpoint of improving the texture of the obtained printed article, the method of applying the ink is preferably an ink jet method. That is, it is preferable to apply the ink composition by an ink jet recording method. The ink jet recording method is as described in the section of the pretreatment liquid applying step.

An amount of droplets of the ink jetted from an ink jet head is preferably 1 pL (picoliter) to 150 pL, more preferably 2 pL to 120 pL, and particularly preferably 20 pL to 60 pL. The amount of droplets jetted means a volume of ink jetted from one nozzle at one time by the ink jet recording method.

From the viewpoint of texture, an application amount of the ink is preferably 10 g/m² to 30 g/m², and more preferably 15 g/m² to 25 g/m².

A resolution of the ink in jetting is preferably 200 dpi or more × 200 dpi or more, and more preferably 400 dpi or more × 400 dpi or more and 1200 dpi or less × 1200 dpi or less.

### (Heat treatment step)

It is preferable that the textile printing method according to the present disclosure includes a step of heating the fabric to which the ink for textile printing is applied at a temperature of 100°C or higher, after the ink applying step.

Examples of a thermal treatment device include a heating drum, hot air, an infrared lamp, a heating oven, a heating plate, a heat press, and a hot plate. The heating temperature is more preferably 100°C to 180°C and still more preferably 120°C to 170°C. In addition, a heating time is preferably 5 seconds to 200 seconds and more preferably 30 seconds to 160 seconds.

The textile printing method according to the present disclosure may include a step other than the above-described steps.

However, it is preferable that the textile printing method according to the present disclosure does not comprise a step of washing the fabric to which the ink for textile printing is applied, after the ink applying step.

The textile printing method according to the present disclosure is performed using the pretreatment liquid and the ink described above, and since the above-described pretreatment liquid contains the ammonium compound (1), fixing property of the pigment to the fabric is excellent, and it is not necessary to wash the fabric to which the ink is applied.

### Examples

Hereinafter, the present disclosure will be described in more detail using Examples. However, the present disclosure is not limited to the following examples as long as it does not exceed the gist of the present invention.

### [Preparation of pretreatment liquid]

The following components were mixed and stirred for 60 minutes to prepare a pretreatment liquid.
· Quaternary ammonium salt: quaternary ammonium salt described in Tables 1 to 6 ... content described in Tables 1 to 6
· Organic solvent: organic solvent described in Tables 1 to 6 ... content described in Tables 1 to 6
· Surfactant: product name "OLFINE E1010", manufactured by Nissin Chemical Co., Ltd. ... 1.0% by mass
· Water: remaining amount set such that the total amount of the pretreatment liquid reached 100% by mass

### [Preparation of ink]

· APD4000 Black (manufactured by Fujifilm Imaging Colorants) ... 4% by mass as a content of the pigment
· Organic solvent: organic solvent described in Tables 1 to 6 ... content described in Tables 1 to 3
· Resin particles: resin particles described in Tables 1 to 6 ... content described in Tables 1 to 6
· Surfactant: product name "OLFINE E1010", manufactured by Nissin Chemical Co., Ltd. ... 2.2% by mass
· Surfactant: product name "BYK345", manufactured by BYK-Chemie GmbH ... 0.1% by mass
· Polyvinylpyrrolidone: product name "PVPK15", manufactured by Tokyo Chemical Industry Co., Ltd. ... 0.15% by mass
· Colloidal silica dispersion liquid: product name "ST-XS", manufactured by Nissan Chemical Corporation ... 0.05% by mass as a content of colloidal silica particles
· Water: remaining amount set such that the total amount of the ink reached 100% by mass

Hereinafter, the ammonium salt and the organic solvent, used for the preparation of the pretreatment liquid, and the organic solvent and the resin particles, used for the preparation of the ink, will be described. In a case where the pretreatment liquid contains the quaternary ammonium cation (1), since the quaternary ammonium salt is used as a raw material, details of the raw material are shown.

In the tables, "-" is shown for components which are not contained.

### [Salt of quaternary ammonium cation (1)]

· DADMAC: diallyldimethylammonium chloride
· Choline chloride
· ABDMAC: N-(2-acryloyloxyethyl)-N-benzyl-N,N-dimethylammonium chloride
· Compound 1: didecyldimethylammonium chloride (product name "didecyldimethylammonium chloride", manufactured by Sigma-Aldrich Co., LLC)

### [Cationic compound]

The following compounds do not correspond to the salt of the quaternary ammonium cation (1).
· PolyDADMAC: diallyldimethylammonium chloride polymer (product name "PAS-H1L", manufactured by NITTOBO MEDICAL CO.,LTD.)
· Compound 2: lauryltrimethylammonium chloride (product name "COTAMINE 24P", manufactured by Kao Corporation)

In Table 3, the cationic compound is also described in the column of the quaternary ammonium cation.

### [Organic solvent]

· PG: propylene glycol
· 1,2-BD: 1,2-butanediol
· 1,3-PD: 1,3-propanediol
· EG: ethylene glycol
· DPGmME: dipropylene glycol monomethyl ether

### [Resin particles]

Urethane A: aqueous dispersion liquid of urethane resin particles (product name "NeoRez R940", manufactured by Kusumoto Chemicals, Ltd.)
Urethane B: aqueous dispersion liquid of urethane resin particles (product name "SUPERFLEX 460", manufactured by DKS Co., Ltd.)
Urethane C: aqueous dispersion liquid of urethane resin particles (product name "EVAFANOL HA-15", manufactured by NIKKA CHEMICALS CO., LTD.)
Styrene-butadiene: aqueous dispersion liquid of styrene-butadiene resin particles (product name "Rovene 4170", manufactured by Mallard Creek Polymers)

### <Ink jet printing>

A fabric was subjected to pretreatment using the pretreatment liquid prepared in Examples and Comparative Examples. The ink prepared in Examples and Comparative Examples was applied onto the pretreated fabric, and the fabric was subjected to a heat treatment to obtain a printed article. Details thereof are as follows.

As the fabric, a long cloth (15 cm × 4 cm) made of 100% cotton (product name "Cotton D5005", manufactured by Akahori Sangyo Co., Ltd.) was prepared.

As an ink jet recording device, a device equipped with an ink jet head (product name "StarFire SG-1024SA", manufactured by Fujifilm Dimatix, Inc.) and an ink circulation pump was prepared. The fabric was fixed on a stage as a recording medium. The pretreatment liquid and the ink composition were respectively filled in an ink tank connected to the ink jet head. The ink jet head was disposed in a line shape such that nozzles were arranged in a direction orthogonal to a movement direction of the stage. Jetting conditions of the pretreatment liquid and the ink were set such that an amount of droplets jetted was 60 pL, a jetting frequency was 10 kHz, and a resolution was 400 dpi × 400 dpi. The ink circulation pump was operated such that the pretreatment liquid and the ink circulated between the ink tank and the ink jet head.

First, the pretreatment liquid was jetted onto the entire surface of the recording medium to record a solid image. The fabric was dried with hot air at 60°C for 10 seconds, and the ink was jetted to record an image, thereby obtaining a colored fabric.

The obtained colored fabric was subjected to a heat treatment using a heat press machine (a tabletop automatic flat heat press, model AF-54TEN, manufactured by Asahi Fiber Machinery Co., Ltd.) under conditions of 160°C and 120 seconds to obtain a printed article.

### <Evaluation>

The following evaluations were performed using the above-described printed article. The evaluation methods were as follows.

### (Washing fastness)

The printed article was subjected to evaluation based on ISO 105-C06:2010 B1S. In a case of corresponding to a plurality of evaluation standards, the highest evaluation was adopted.
AA: both contamination and discoloration were 4th grade or 5th grade.
A: both contamination and discoloration were 4th grade or higher.
B: both contamination and discoloration were 3rd grade or 4th grade or higher.
C: both contamination and discoloration were 3rd grade or higher.
D: any of contamination or discoloration was 2nd grade.

### (Image quality)

A character image shown in FIG. 1 was recorded in sizes of 10 pt, 12 pt, 14 pt, and 16 pt. The recorded character image was visually observed to evaluate image quality.
AA: there was no collapse of the character image in all sizes of 10 pt, 12 pt, 14 pt, and 16 pt.
A: there was no collapse of the character image at sizes of 12 pt, 14 pt, and 16 pt, but there was collapse at a size of 10 pt.
B: there was no collapse of the character image at sizes of 14 pt and 16 pt, but there was collapse at sizes of 10 pt and 12 pt.
C: there was no collapse of the character image at a size of 18 pt, but there was collapse at sizes of 10 pt, 12 pt, and 14 pt.
D: there was collapse of the character image in all sizes of 10 pt, 12 pt, 14 pt, and 16 pt.

### (Texture)

A rectangular sample for evaluation, having a length (long side) of 150 mm and a width (short side) of 50 mm, was cut out from the obtained printed article. As a jig for evaluation, a stainless steel plate having a length (long side) of 200 mm, a width (short side) of 100 mm, and a thickness of 1 mm was prepared. The jig for evaluation stood such that a short side direction was a vertical direction and a long side direction was a horizontal direction. Next, a center portion (that is, a center line) of the sample for evaluation in the longitudinal direction was placed on the long side of the jig for evaluation facing in a horizontal direction, and both sides (both end part sides) of the sample for evaluation in the longitudinal direction were hung.

In this state, a linear distance between one end part and the other end part of the sample for evaluation in the longitudinal direction was measured, and texture of the printed article was evaluated based on the following evaluation standard. In the evaluation, as the sample for evaluation was softer (that is, the texture was more excellent), both sides of the sample for evaluation in the longitudinal direction hung down due to the self-weight (that is, the sample for evaluation was bent), and as a result, the linear distance between one end part and the other end part of the sample for evaluation in the longitudinal direction was shortened.
AA: the linear distance was less than 40 mm.
A: the linear distance was 40 mm or more and less than 55 mm.
B: the linear distance was 55 mm or more and less than 70 mm.
C: the linear distance was 70 mm or more and less than 85 mm.
D: the linear distance was 85 mm or more.

### (Rub resistance)

The printed article was subjected to evaluation based on ISO 105-X12:2001 (wet).
A: 3rd grade or 4th grade or higher
B: 3rd grade
C: 2nd grade or 3rd grade
D: 2nd grade or lower

The evaluation results are shown in Tables 1 to 6.

In Table 6, the compound 2 did not correspond to the quaternary ammonium cation (1), but a group corresponding to R¹ and R² in Formula (1) was described.

**[Table 1]**

| | | | Example 1 | Example 2 | Example 3 | Example 4 | Example 5 | Example 6 | Example 7 |
|---|---|---|---|---|---|---|---|---|---|
| Pretreatment liquid | Quaternary ammonium cation (1) | Raw material | DADMAC | Choline chloride | Choline chloride | Choline chloride | Choline chloride | Choline chloride | Choline chloride |
| | | R¹ | Allyl group | Methyl group | Methyl group | Methyl group | Methyl group | Methyl group | Methyl group |
| | | R² | Allyl group | Hydroxyethyl group | Hydroxyethyl group | Hydroxyethyl group | Hydroxyethyl group | Hydroxyethyl group | Hydroxyethyl group |
| | | Formula weight | 126.2 | 104.2 | 104.2 | 104.2 | 104.2 | 104.2 | 104.2 |
| | | Content | 15 | 15 | 10 | 6 | 10 | 5 | 10 |
| | Organic solvent | PG | 10 | 10 | 10 | 10 | 30 | 30 | 10 |
| Ink | Organic solvent | PG | 20 | 20 | 20 | 20 | 25 | 25 | 20 |
| | | DPGmME | 5 | 5 | 5 | 5 | 5 | 5 | - |
| | Resin particles | Type | Urethane A | Urethane A | Urethane A | Urethane A | Urethane A | Urethane A | Urethane A |
| | | Content | 7 | 7 | 7 | 7 | 7 | 7 | 7 |
| A/B | | | 40 | 48 | 32 | 19 | 17 | 9 | 32 |
| Evaluation | | Washing fastness | AA | AA | A | B | A | A | A |
| | | Image quality | AA | AA | A | B | B | C | A |
| | | Texture | AA | AA | A | A | B | B | A |
| | | Rub resistance | A | A | A | A | B | B | C |

**[Table 2]**

| | | | Example 8 | Example 9 | Example 10 | Example 11 | Example 12 |
|---|---|---|---|---|---|---|---|
| Pretreatment liquid | Quaternary ammonium cation (1) | Raw material | Choline chloride | Choline chloride | DADMAC | DADMAC | DADMAC |
| | | R¹ | Methyl group | Methyl group | Allyl group | Allyl group | Allyl group |
| | | R² | Hydroxyethyl group | Hydroxyethyl group | Allyl group | Allyl group | Allyl group |
| | | Formula weight | 104.2 | 104.2 | 126.2 | 126.2 | 126.2 |
| | | Content | 6 | 10 | 15 | 15 | 15 |
| | Organic solvent | PG | 2 | 40 | 10 | 10 | 10 |
| Ink | Organic solvent | PG | 20 | 25 | 20 | 20 | 20 |
| | | DPGmME | 5 | 5 | 5 | 5 | 5 |
| | Resin particles | Type | Urethane A | Urethane A | Styrene-butadiene | Urethane B | Urethane C |
| | | Content | 7 | 7 | 7 | 7.5 | 7.5 |
| A/B | | | 26 | 15 | 40 | 40 | 40 |
| Evaluation | | Washing fastness | B | A | AA | AA | AA |
| | | Image quality | B | C | AA | AA | AA |
| | | Texture | A | B | B | AA | AA |
| | | Rub resistance | B | B | A | A | A |

**[Table 3]**

| | | | Example 13 | Example 14 | Example 15 | Example 16 | Example 17 |
|---|---|---|---|---|---|---|---|
| Pretreatment liquid | Quaternary ammonium cation (1) | Raw material | DADMAC | DADMAC | DADMAC | DADMAC | ABDMAC |
| | | R¹ | Allyl group | Allyl group | Allyl group | Allyl group | Acryloyloxyethyl group |
| | | R² | Allyl group | Allyl group | Allyl group | Allyl group | Benzyl group |
| | | Formula weight | 126.2 | 126.2 | 126.2 | 126.2 | 234.3 |
| | | Content | 15 | 15 | 15 | 15 | 15 |
| | Organic solvent | 1,2-BD | 10 | - | - | - | - |
| | | 1,3-PD | - | 10 | - | - | - |
| | | EG | - | - | 15 | - | - |
| Ink | Organic solvent | PG | 20 | 20 | 20 | 20 | 20 |
| | | DPGmME | 5 | 5 | 5 | 5 | 5 |
| | Resin particles | Type | Urethane A | Urethane A | Urethane A | Urethane A | Urethane A |
| | | Content | 7 | 7 | 7 | 7 | 7 |
| A/B | | | 40 | 40 | 59 | 59 | 32 |
| Evaluation | | Washing fastness | AA | AA | AA | A | A |
| | | Image quality | AA | AA | AA | A | A |
| | | Texture | AA | AA | AA | A | A |
| | | Rub resistance | A | A | A | B | A |

**[Table 4]**

| | | | Example 18 | Example 19 | Example 20 | Example 21 | Example 22 | Example 23 |
|---|---|---|---|---|---|---|---|---|
| Pretreatment liquid | Quaternary ammonium cation (1) | Raw material | DADMAC | DADMAC | DADMAC | DADMAC | DADMAC | DADMAC |
| | | R¹ | Allyl group | Allyl group | Allyl group | Allyl group | Allyl group | Allyl group |
| | | R² | Allyl group | Allyl group | Allyl group | Allyl group | Allyl group | Allyl group |
| | | Formula weight | 126.2 | 126.2 | 126.2 | 126.2 | 126.2 | 126.2 |
| | | Content | 15 | 15 | 15 | 15 | 15 | 15 |
| | Organic solvent | 1,2-BD | - | - | - | - | - | 10 |
| Ink | Organic solvent | PG | 20 | 20 | 20 | 20 | 20 | 4 |
| | | DPGmME | - | - | - | - | - | 21 |
| | | PGmME | 5 | - | - | - | - | - |
| | | DEGmEE | - | 5 | - | - | - | - |
| | | TPGmME | - | - | 5 | - | - | - |
| | | DEGmBE | - | - | - | 5 | 1 | - |
| | Resin particles | Type | Urethane A | Urethane A | Urethane A | Urethane A | Urethane A | Urethane A |
| | | Content | 7 | 7 | 7 | 7 | 7 | 7 |
| A/B | | | 59 | 59 | 59 | 59 | 59 | 85 |
| Evaluation | | Washing fastness | A | A | A | A | A | AA |
| | | Image quality | A | A | A | A | A | B |
| | | Texture | A | A | A | A | A | A |
| | | Rub resistance | B | A | A | A | B | A |

**[Table 5]**

| | | | Comparative Example 2 | Comparative Example 4 | Comparative Example 5 |
|---|---|---|---|---|---|
| Pretreatment liquid | Quaternary ammonium cation (1) | Raw material | Compound 1 | Choline chloride | DADMAC |
| | | R¹ | Decyl group | Methyl group | Allyl group |
| | | R² | Decyl group | Hydroxyethyl group | Allyl group |
| | | Formula weight | 327 | 104.2 | 126.2 |
| | | Content | 15 | 3 | 33 |
| | Organic solvent | PG | 10 | 15 | 10 |
| | | 1,2-BD | - | - | - |
| | | 1,3-PD | - | - | - |
| | | EG | - | - | - |
| Ink | Organic solvent | PG | 20 | 15 | 20 |
| | | DPGmME | 5 | 5 | 5 |
| | Resin particles | Type | Urethane A | Urethane A | Urethane A |
| | | Content | 7 | 7 | 7 |
| A/B | | | 15 | 10 | 87 |
| Evaluation | | Washing fastness | B | B | AA |
| | | Image quality | C | D | AA |
| | | Texture | D | D | D |
| | | Rub resistance | C | B | C |

**[Table 6]**

| | | | Comparative Example 1 | Comparative Example 3 |
|---|---|---|---|---|
| Pretreatment liquid | Cationic compound | Raw material | PolyDADMAC | Compound 2 |
| | | R¹ | - | Methyl group |
| | | R² | - | Lauryl group |
| | | Formula weight | 8500 | 327 |
| | | Content | 15 | 15 |
| | Organic solvent | PG | 10 | 10 |
| Ink | Organic solvent | PG | 20 | 20 |
| | | DPGmME | 5 | 5 |
| A/B | | | 40 | 15 |
| Evaluation | | Washing fastness | C | B |
| | | Image quality | C | D |
| | | Texture | D | A |
| | | Rub resistance | D | A |

As shown in Tables 1 to 6, in Examples 1 to 23, since the pretreatment liquid contained the quaternary ammonium cation (1) having a formula weight of 100 to 300, and the content of the quaternary ammonium cation represented by Formula (1) was 5% by mass to 30% by mass with respect to the total amount of the pretreatment liquid, a printed article having excellent washing fastness, favorable texture, and excellent image quality could be obtained.

In Comparative Example 1, it was found that the printed article had a deteriorated texture because the quaternary ammonium cation (1) was not contained.

In Comparative Example 2, it was found that the printed article had a deteriorated texture because the content of the quaternary ammonium cation (1) was more than 300.

In Comparative Example 3, it was found that the printed article had a deteriorated image quality because the quaternary ammonium cation included the group having more than 10 carbon atoms.

In Comparative Example 4, it was found that the printed article had a deteriorated image quality and texture because the content of the quaternary ammonium cation (1) was less than 5% by mass.

In Comparative Example 5, it was found that the printed article had a deteriorated texture because the content of the quaternary ammonium cation (1) was more than 30% by mass.

In Example 4, it was found that the content of the alkanediol in the pretreatment liquid was 3% by mass or more, and thus the printed article had excellent rub resistance as compared with Example 8.

In Example 3, it was found that the content of the alkanediol in the pretreatment liquid was 30% by mass or less, and thus the printed article had excellent image quality, texture, and rub resistance as compared with Example 9.

In Example 21, it was found that the content of the alkylene glycol alkyl ether in the ink was 2% by mass or more, and thus the printed article had excellent rub resistance as compared with Example 22.

In Example 13, it was found that the content of the alkylene glycol alkyl ether in the ink was 20% by mass or less, and thus the printed article had excellent image quality and texture as compared with Example 23.

In Examples 1, 11, and 12, it was found that the ink contained urethane resin particles, and thus the printed article had excellent texture as compared with Example 10.

The disclosure of Japanese Patent Application No. 2023-168522 filed on September 28, 2023 is incorporated in the present specification by reference. In addition, all documents, patent applications, and technical standards described in the present specification are incorporated herein by reference to the same extent as in a case of being specifically and individually noted that individual documents, patent applications, and technical standards are incorporated by reference.

## Claims

1. A pretreatment liquid for textile printing, comprising:
a quaternary ammonium cation represented by Formula (1), having a formula weight of 100 to 300;
wherein a content of the quaternary ammonium cation represented by Formula (1) is 5% by mass to 30% by mass with respect to a total amount of the pretreatment liquid for textile printing, and
the pretreatment liquid for textile printing is used before image recording on a fabric using an ink for textile printing, containing a pigment, 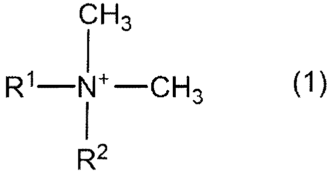
in Formula (1), both R¹ and R² are a group having 10 or less carbon atoms.

2. The pretreatment liquid for textile printing according to claim 1,
wherein R¹ and R² are each independently an unsubstituted alkyl group, a substituted alkyl group, a vinyl group, an aryl group, an allyl group, an alkyl ester group, a (meth)acryloyl group, or an amino group, each having 10 or less carbon atoms.

3. The pretreatment liquid for textile printing according to claim 1, further comprising:
an alkanediol,
wherein a content of the alkanediol is 3% by mass to 30% by mass with respect to the total amount of the pretreatment liquid for textile printing.

4. The pretreatment liquid for textile printing according to claim 1,
wherein the quaternary ammonium cation represented by Formula (1) is at least one selected from the group consisting of a diallyldimethylammonium cation, a (2-hydroxyethyl)trimethylammonium cation, and an N-(2-acryloyloxyethyl)-N-benzyl-N,N-dimethylammonium cation.

5. The pretreatment liquid for textile printing according to claim 1,
wherein the formula weight of the quaternary ammonium cation represented by Formula (1) is 100 to 200.

6. An ink set for textile printing, comprising:
the pretreatment liquid for textile printing according to any one of claims 1 to 5; and
an ink for textile printing, containing a pigment.

7. The ink set for textile printing according to claim 6,
wherein the ink for textile printing contains an alkanediol, and
a content of the alkanediol is 3% by mass to 30% by mass with respect to a total amount of the ink for textile printing.

8. The ink set for textile printing according to claim 6,
wherein at least one of the pretreatment liquid for textile printing or the ink for textile printing contains an alkanediol, and
in a case where the pretreatment liquid for textile printing and the ink for textile printing have the same mass,
an effective cation amount in the pretreatment liquid for textile printing is defined as A mmol/L, and
a total content of the alkanediol contained in the pretreatment liquid for textile printing and the ink for textile printing is defined as B% by mass,
a ratio of A to B is 15 or more.

9. The ink set for textile printing according to claim 6,
wherein the ink for textile printing further contains an alkylene glycol alkyl ether, and
a content of the alkylene glycol alkyl ether is 2% by mass to 20% by mass with respect to a total amount of the ink for textile printing.

10. The ink set for textile printing according to claim 6,
wherein the ink for textile printing further contains urethane resin particles.

11. The ink set for textile printing according to claim 6,
wherein the pigment is dispersed by a polymer having a crosslinking structure.

12. A textile printing method comprising:
a pretreatment liquid applying step of applying the pretreatment liquid for textile printing, which is contained in the ink set for textile printing according to claim 6, onto a fabric; and
an ink applying step of applying the ink for textile printing, which is contained in the ink set for textile printing according to claim 6, onto the fabric to which the pretreatment liquid for textile printing is applied.

13. The textile printing method according to claim 12,
wherein, in the pretreatment liquid applying step, the pretreatment liquid for textile printing is applied by an ink jet recording method.

14. The textile printing method according to claim 13,
wherein the ink jet recording method is performed in a single pass.

15. The textile printing method according to claim 12, further comprising:
a step of heating the fabric to which the ink for textile printing is applied at a temperature of 100°C or higher, after the ink applying step.

16. The textile printing method according to claim 12,
wherein the textile printing method does not comprise a step of washing the fabric to which the ink for textile printing is applied, after the ink applying step.

17. The textile printing method according to claim 12,
wherein the fabric is in roll form and is made of cotton or a cotton-polyester blend.
